(19) Europäisches Patentamt
European Patent Office
Office européen des brevets

(11) EP 0 474 597 B1

# (12) EUROPÄISCHE PATENTSCHRIFT

(45) Veröffentlichungstag und Bekanntmachung des Hinweises auf die Patenterteilung:
**06.08.1997 Patentblatt 1997/32**

(51) Int. Cl.$^6$: **A61K 31/075**

(21) Anmeldenummer: **91810685.7**

(22) Anmeldetag: **27.08.1991**

(54) **Arachidonsäurestoffwechselhemmende Diphenylverbindungen und ihre Verwendung in pharmazeutischen Zusammensetzungen**

Diphenyl compounds as inhibitors of the arachidonic acid pathway and their use in pharmaceutical compositions

Composés diphényls inhibiteurs du métabolisme de l'acide arachidonique et leur utilisation dans des compositions pharmaceutiques

(84) Benannte Vertragsstaaten:
**AT BE CH DE DK ES FR GB GR IT LI LU NL SE**

(30) Priorität: **05.09.1990 CH 2875/90**

(43) Veröffentlichungstag der Anmeldung:
**11.03.1992 Patentblatt 1992/11**

(73) Patentinhaber: **Ciba Specialty Chemicals Holding Inc.**
**4057 Basel (CH)**

(72) Erfinder:
- **Schewe, Tankred, Prof. Dr.**
**O-1140 Berlin (DE)**
- **Luther, Helmut, Dr.**
**W-7800 Freiburg (DE)**
- **Jordanov, Dentscho, Dr.**
**O-1130 Berlin (DE)**

(56) Entgegenhaltungen:
**EP-A- 0 220 890** **EP-A- 0 271 332**
**GB-A- 2 148 116** **GB-A- 2 207 604**
**US-A- 4 512 987** **US-A- 4 735 802**

- **CLINICAL TRIALS JOURNAL, Band 24, Nr. 3, 1987, Seiten 222-226, Dr. Stuart Phillips, Sutton, Surrey, GB; P.D. PIGATTO et al.: "Halometasone/triclosan cream: (Sicorten Plus) - A study of Clinical and allergological behaviour"**
- **CONTACT DERMATITIS, Band 18, Nr. 4, 1988, Seiten 243-244; B. STEINKJER et al.: "Contact dermatitis from triclosan (Irgasan DP 300)"**
- **J. PERIODONT. RES., Band 24, Nr. 1, 1989, Seiten 75-80; C.A. SAXTON et al.: "The effect of a dentifrice containing zinc citrate and triclosan on developing gingivitis"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 474 597 B1

## Beschreibung

Die Erfindung betrifft die Verwendung von pharmazeutischen Zusammensetzungen mit lipoxygenase- und prostaglandin H-synthasehemmende Wirkstoffen zur vorzugsweise topischen Applikation in der Human- und Veterinärmedizin. Sie dienen insbesondere zur Behandlung entzündlicher Hauterkrankungen nichtmikrobieller Genese wie z.B. Hauterythemen.

Zur topischen Behandlung von inflammatorischen und allergischen Erkrankungen werden vorrangig Glukokortikoide eingesetzt. Es ist allgemein bekannt, dass diese unerwünschte Nebenwirkungen haben.

Nichtsteroidale entzündungshemmende Arzneimittel, die Wirkstoffe wie z.B. Ketoprofen, BW 755 c, Piroxicam, Diclofenac bzw. Indomethazin enthalten, können aufgrund ihrer unzureichenden Hautpenetration (siehe z.B. G.B. Kasting et al., Pharmacol. Scin., Vol. 1, pp. 138-153, Karger, Basel 1987) nicht wirkungsvoll lokal begrenzt, sondern nur systemisch angewendet werden. Gut hautpenetrierende 2-Hydroxy-diphenyläther, 2-Hydroxy-diphenylmethane und 2-Hydroxy-diphenylthioäther als Bestandteile von pharmazeutischen Zubereitungen wurden bisher nur für eine antimikrobielle Wirksamkeit beansprucht (DRP 568.944, CH 428.758, DE 1.216.882) bzw. eingesetzt [z.B. H. Abdel Aal et al., J. Int. Med. Res. 15, 383 (1987)].

Die Behandlung von entzündlichen Erkrankungen durch die genannten Verbindungen, die auf einer Störung der endogenen Stoffwechselregulation beruhen, sind nicht bekannt.

Ziel der Erfindung ist die Bereitstellung von pharmazeutischen Zusammensetzungen mit pharmakologisch wertvollen, antiinflammatorischen Eigenschaften bei insbesondere lokaler und/oder inhalativer Anwendung.

Aufgabe der Erfindung ist die Entwicklung von pharmazeutischen Zusammensetzungen mit antiinflammatorischen Eigenschaften, die insbesondere auf der Hemmung der Oxygenierungsreaktionen des Arachidonsäurestoffwechsels beruhen und bei lokaler Anwendung keine unerwünschte Nebenwirkungen zeigen.

Ueberraschenderweise wurde gefunden, dass an und für sich bekannte 2-Hydroxy-diphenyläther der allgemeinen Formel

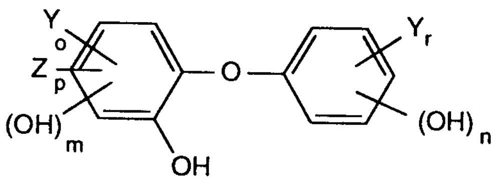

(I),

worin Y Chlor oder Brom, Z $SO_2H$, $NO_2$, oder $C_1$-$C_4$-Alkyl bedeuten, und r = 0 bis 3, o = 0 bis 3, p = 0 oder 1, m = 0 oder 1 und n= 0 oder 1 bedeutet,
ausgeprägte antiinflammatorische Wirkungen zeigen.

Die antiinflammatorische Wirkung wird in zellulären in vitro und un tierexperimentellen in vitro- und in vivo-Untersuchungen bestätigt.

Gegenstand der vorliegenden Erfindung ist die Verwendung einer Verbindung der Formel (I) zur Herstellung pharmazeutischer Präparate für die Behandlung von inflammatorischen Erkrankungen.

Besonders interessant haben sich Verbindungen der Formel (I) erwiesen, worin Y Chlor oder Brom bedeuten, und m = 0, n = 0 oder 1, o = 1 oder 2, r = 1 oder 2 und p = 0 sind.

Ganz besonders interessant haben sich Verbindungen der Formel (I) erwiesen, worin Y Chlor bedeuten, und m = 0, n = 0, o = 1, r = 2 und p = 0 sind.

Die Verbindungen der Formel hemmen (I) in niedrigen Konzentrationen die gereinigte erythroide 15-Lipoxygenase von Kaninchen [nach Schewe, T.; Kühn, H; Rapoport, S.M.; in: Benedetto, C.; McDonald-Gibson, R.C.; Nigam, S.; Slater, T.F. (Eds.): Prostaglandins and related substances, pp. 229, IRL Press, Oxford, Washington 1987](Beispiel 4) sowie die Prostaglandin H-Synthase aus Schafssamenblasen [nach Van der Ouderaa, F.J.G. et al., Methods Enzymol. 86, 60 (1982)] (Beispiel 5).

Eicosanoide, die durch Lipoxygenasen oder Prostaglandin H-Synthase aus Arachidonsäure in Säugetierzellen gebildet werden, spielen eine Schlüsselrolle in der Pathogenese des Asthma bronchiale [Dahlén, S.-R. et al., Respiration 50, Suppl. 2, 22 (1986)], der Psoriasis [Greaves, M.W.; in Piper, P.J. (Ed.): The Leukotrienes: Their Biological Significance, pp. 175, Raven Press, New York, 1986], UV-Lichtinduzierter Dermatitis [Søndergaard, J. et al., Photodermatol. 359, 66 (1985)] und einer Vielzahl von allergischen Krankheitsbildern [Stenson, W.F. et al. Clin. Rev. Allergy 1, 369 (1983); Parker, C.W. in: Ring, J. (Ed): New Trends in Allergy II, pp. 137, Springer-Verlag, Heidelberg 1985]. Demzufolge sind die Verbindungen I erfindungemäss direkt oder als Bestandteil galenischer Zubereitungen zusammen mit einem oder mehreren pharmazeutisch verwendbaren Trägermaterialien kurativ für alle Formen von inflammatorischen Erkrankungen nichtmikrobieller Genese wirksam.

Als galenische Formen, die die Verbindungen der Formel (I) enthalten, sind insbesondere Dareichungsformen für

die lokale (topische) und inhalatorische Behandlung, wie Emulsionen, Salben, Gele, Sprays, Puder u.a. zu verstehen. Verbindungen der Formel (I) können auch in Liposomen enthalten sein oder mit üblichen Trägerstoffen und/oder Penetrationsbeschleunigern wie z.B. Harnstoff, Propylenglykol, Oleinsäure u.a. [siehe auch Barry, B.W. in: Schroot, B.; Schaefer, H. (Eds.): Pharmacol. Skin., Vol. 1, pp. 121, Karger, Basel 1987] in pharmakologischen Zubereitungen eingesetzt werden. Verbindungen der Formel (I) und die in erfindungsgemässen pharmazeutischen Zubereitungen sind im allgemeinen in Mengen von 0,01 bis 15, vorzugsweise 0,1 bis 4 Gew.% der Gesamtmischung vorhanden. Die erfindungsgemässen pharmazeutischen Zubereitungen können für die Behandlung der angeführten Erkrankungen ausser den Verbindungen der Formel (I) auch weitere pharmazeutische Wirkstoffe enthalten, z.B. antiinflammatorische Wirkstoffe.

Beispiel 1:

Hemmung der Aktivität der 15-Lipoxygenase aus Kanincherenretikulozyten

Die Lipoxygenase aus Kaninchenretikulozyten wird nach dem in der Literatur beschriebenen Verfahren in elektrophoretisch und immunologisch reiner Form erhalten [S.M. Rapoport et al., Eur. J. Biochem. 96, 545 (1979)]. Die Bestimmung der Lipoxygenaseaktivität erfolgt bei 25°C über die amperometrische Messung des $O_2$-Verbrauchs mittels einer Clark-Elektrode in folgendem System: 0,1 M Kaliumphosphat pH 7,4 mit 0,2% Natriumcholat und 0,53 mM Linolsäure. Die Enzymkonzentration beträgt im Messansatz 25 nM. Die zu prüfenden Substanzen werden in Methylglykol (frisch destilliert) gelöst zugesetzt. Die Verdünnungen der Verbindungen werden so gewählt, dass die Endkonzentrationen an Methylglykol im Vorinkubationssatz 2 % nicht überstieg; unter diesen Bedingungen treten keine nennenswerte Hemmungen in den Kontrollansätzen auf. Die Enzymreaktion wird durch Zusatz von Natriumcholat und Linolsäure gestartet. Durch Variation der Wirkstoffkonzentration wird die Titrationskurve der Hemmung und daraus die erforderliche Konzentration für eine 50 %ige Hemmung ermittelt.

Die Ergebnisse sind in Tabelle 1 dargestellt

Tabelle 1

| | 15-LOX $IC_{50}$ (µM) |
|---|---|
| - 2,4,4'-Trichlor-2'-hydroxy-diphenylether (Triclosan) | 2,4 |
| - 2,2'-Dihydroxy-5,5'-dichlor-diphenylsulfid (Fenticlor) | 1,4 |
| - 2,2'-Dihydroxy-3,3',5,5'-tetrachlor-diphenylmethan | 9,5 |
| - 2,2'-Dihydroxy-3,3',5,5'-tetrabrom-diphenylmethan | 2,8 |
| - 2,2'-Dihydroxy-3,3'-dichlor-5,5'-dibrom-diphenylmethan | 3,8 |
| - 4,4'-Dihydroxy-3,3',5,5'-tetrabrom-diphenylmethan | 5,0 |
| - 4,4'-Dihydroxy-3,3'-dibrom-5,5'-dichlor-diphenylmethan | 10,5 |
| - 2,2'6,6'-Tetrahydroxy-3,3',5,5'-tetrachlordiphenylmethan | 3,6 |
| - 2,2'-Dihydroxy-3,3',5,5',6,6'-hexachlor-diphenylmethan | 3,5 |
| - 2-Benzyl-4-chlorphenol (Chlorophen) | 2,0 |
| - 4-Chlor-2-methyl-6-benzylphenol | 2,5 |
| - 2-Chlor-6-methyl-4-benzylphenol | 28,0 |

Beispiel 2:

Hemmung der Aktivität der Prostaglandin H-Synthase aus Schafssamenblasen

Prostaglandin H-Synthase aus Schafssamenblasen wird nach dem in der Literatur beschriebenen Verfahren Van der Oudaraa, F.J.G.; Buytenhek, M.; Methods Enzymol. 86, 60 (1982) erhalten.

Die Bestimmung der Enzymaktivität erfolgt bei 25°C durch polarografische Messung des Sauerstoffverbrauchs mit einer Clark-Elektrode.

Der Messansatz besteht aus 400 µg $ml^{-1}$ Prostaglandin H-Synthase in 0,1 M Tris-HCl-Puffer pH = 8,0, 5 mM Tryptophan, 1 µM Haemin und 0,124 mM Arachidonsäure. Die zu prüfenden Substanzen werden in Methylglykol gelöst und 10 Minuten bei 25°C mit dem Enzym in Abwesenheit von Arachidonsäure inkubiert. Die Enzymreaktion wird mit Arachidonsäure gestartet.

Durch Variation der Wirkstoffkonzentration werden die Titrationskurve der Hemmung und daraus die erforderliche Konzentration für eine 50 %ige Hemmung ermittelt.

Die Ergebnisse sind in Tabelle 2 dargestellt

Tabelle 2

| | PG-H-Synthase $IC_{50}$ (µM) |
|---|---|
| - 2,4,4'-Trichlor-2'-hydroxy-diphenylether (Triclosan) | 23,0 |
| - 2,2'-Dihydroxy-5,5'-dichlor-diphenylsulfid (Fenticlor) | 15,5 |
| - 2,2'-Dihydroxy-3,3',5,5'-tetrachlor-diphenylmethan | 60,0 |
| - 2,2'-Dihydroxy-3,3',5,5'-tetrabrom-diphenylmethan | 45,0 |
| - 2,2'-Dihydroxy-3,3'-dichlor-5,5'-dibrom-diphenylmethan | 28,0 |
| - 4,4'-Dihydroxy-3,3',5,5'-tetrabrom-diphenylmethan | 17,0 |
| - 4,4'-Dihydroxy-3,3'-dibrom-5,5'-dichlor-diphenylmethan | 180,0 |
| - 2,2',6,6'-Tetrahydroxy-3,3',5,5'-tetrachlordiphenylmethan | 17,0 |
| - 2,2'-Dihydroxy-3,3',5,5',6,6'-hexachlor-diphenylmethan | 17,0 |
| - 2-Benzyl-4-chlorphenol (Chlorophen) | 40,0 |
| - 4-Chlor-2-methyl-6-benzylphenol | 30,0 |
| - 2-Chlor-6-methyl-4-benzylphenol | 35,0 |

Die nachfolgenden Beispiele illustrieren die oben beschriebene Erfindung ohne sie einzuschränken. Temperaturen sind in Celsiusgraden angegeben-

<u>Präparatebeispiel 1:</u> Eine Salbe enthaltend 0,05 % 2,4,4'-Trichlor-2'-hydroxy-diphenylether kann wie folgt hergestellt werden:

<u>Zusammensetzung:</u>

| | |
|---|---|
| Wirkstoff | 0,05 % |
| Vaseline | 45,0 % |
| Paraffinöl | 19,6 % |
| Cetylalkohol | 5,00 % |
| Bienenwachs | 5,00 % |
| Sorbitan-sesquioleat | 5,00 % |
| p-Hydroxybenzoesäureester | 0,20 % |
| Wasser, entmineralisiert, bis zu | 100,00 % |

Die Fettstoffe und Emulgatoren werden zusammengeschmolzen und der Wirkstoff darin gelöst. Das Konservierungsmittel wird in Wasser gelöst, und die Lösung in die Fettschmelze bei erhöhter Temperatur einemulgiert und kaltgerührt.

Präparatebeispiel 2: Eine Crème enthaltend 0,5 % 2,4,4'-Trichlor-2'-hydroxy-diphenylether, kann wie folgt hergestellt werden:

Zusammensetzung:

| | |
|---|---|
| Wirkstoff | 0,5 % |
| Isopropylpalmitat | 8,0 % |
| Cetylpalmitat | 1,5 % |
| Siliconöl 100 | 0,5 % |
| Sorbitanmonostearat | 3,0 % |
| Polysorbat 60 | 3,5 % |
| 1,2-Propylenglykol PH | 20,0 % |
| Acrylsäurepolymerisat | 0,5 % |
| Triäthanolamin | 0,7 % |
| Wasser, entmineralisiert, bis zu | 100,00 % |

Das Acrylsäurepolymerisat wird in einem Gemisch aus entmineralisiertem Wasser und 1,2-Propylenglykol suspendiert. Unter Rühren wird hierauf Triäthanolamin zugegeben, wodurch ein Schleim erhalten wird. Ein Gemisch aus Isopropylpalmitat, Cetylpalmitat, Siliconöl, Sorbitanmonostearat und Polysorbat wird auf ca. 75° erwärmt und darin der Wirkstoff gelöst. Diese Fettphase wird unter Rühren in den gleichfalls auf ca. 75° erwärmten Schleim eingearbeitet und kaltgerührt.

Präparatebeispiel 3: Eine Crème enthaltend 0,05 % 2,4,4'-Trichlor-2'-hydroxy-diphenylether, kann wie folgt hergestellt werden:

Zusammensetzung:

| | |
|---|---|
| Wirkstoff | 0,05 % |
| Cetylpalmitat PH | 2,00 % |
| Cetylalkohol PH | 2,00 % |
| Triglyceridgemisch gesättigter mittelkettiger | |
| Fettsäuren | 5,00 % |
| Stearinsäure | 3,00 % |
| Glycerinstearat PH | 4,00 % |
| Cetomacrogol 1000 | 1,00 % |
| mikrokristalline Cellulose | 0,50 % |
| 1,2-Propylenglykol, dest | 20,00 % |
| Wasser, entmineralisiert, bis zu | 100,00 % |

Cetylalkohol, Cetylpalmitat, das Triglyceridgemisch, Stearinsäure und Glycerinstearat werden zusammengeschmolzen und der Wirkstoff darin gelöst. Die mikrokristalline Cellulose wird in einem Teil des Wassers dispergiert. Im restlichen Teil des Wassers wird Cetomacrogol gelöst und das Propylenglykol sowie der Schleim dazu gemischt. Die Fettphase wird anschliessend unter Rühren zur Wasserphase gegeben und kaltgerührt.

Präparatebeispiel 4: Ein transparentes Hydrogel, enthaltend 0,5 % 2,4,4'-Trichlor-2'-hydroxy-diphenylether, wird wie folgt hergestellt:

Zusammensetzung:

| | |
|---|---|
| Wirkstoff | 0,5 % |
| Propylenglykol | 10,0-20,0 % |
| Isopropanol | 20,0 % |
| Hydroxypropyl-methylcellulose | 2,0 % |
| Wasser | ad 100,00 % |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit dem gequollenen Cellulose-Derivat vermischt und, wenn erwünscht, mit Riechstoffen (0,1 %) versetzt.

Präparatebeispiel 5: Ein transparentes Hydrogel, enthaltend 0,005 % 2,4,4'-Trichlor-2'-hydroxy-diphenylether, wird wie folgt hergestellt:

Zusammensetzung:

| | |
|---|---|
| Wirkstoff | 0,005 % |
| Propylenglykol | 20,0 % |
| Isopropanol | 20,0 % |
| Acrylsäurepolymerisat | 2,0 % |
| Triäthanolamin | 3,0 % |
| Wasser | ad 100,00 % |

Acrylsäurepolymerisat und Wasser werden dispergiert und mit Triäthanolamin neutralisiert. Der Wirkstoff wird in einem Gemisch aus Isopropanol und Propylenglykol gelöst Anschliessend wird die Wirkstofflösung mit einem Gel vermischt, wobei, wenn erwünscht, Riechstoff (0,1 %) zugegeben werden kann.

Präparatebeispiel 6: Ein Schaumspray, enthaltend 0,01 % 2,4,4'-Trichlor-2'-hydroxy-diphenylether, kann wie folgt hergestellt werden:

Zusammensetzung:

| | |
|---|---|
| Wirkstoff | 0,01 % |
| Cetylalkohol PH | 1,70 % |
| Paraffinöl, dickflüssig | 1,00 % |
| Isopropylmyristat | 2,00 % |
| Cetomacrogol 1000 | 2,40 % |
| Sorbitanmonostearat | 1,50 % |
| 1,2-Propylenglykol PH | 5,00 % |
| Methylparaben | 0,18 % |
| Propylparaben | 0,02 % |
| Chemoderm 314 | 0,10 % |
| Wasser, entmineralisiert, bis zu | 100,00 % |

Cetylalkohol, Paraffinöl, Isopropylmyristat, Cetomacrogol und Sorbitanstearat werden zusammengeschmolzen und der Wirkstoff darin gelöst. Methyl- und Propylparaben werden im Propylenglykol gelöst und dem heissen Wasser zugegeben. Die Schmelze und die Lösung werden anschliessend vermischt. Nach dem Abkühlen wird Chemoderm zugeführt und mit Wasser auf das Endgewicht ergänzt.

Abfüllung:

20 ml des Gemisches werden in eine Aluminiumdose eingefüllt. Die Dose wird mit einem Ventil versehen, und das Treibgas wird unter Druck eingefüllt.

Präparatebeispiel 7: Eine Augensalbe enthaltend 1 % 2,4,4'-Trichlor-2'-hydroxy-diphenylether kann folgendermassen hergestellt werden

Zusammensetzung:

| | |
|---|---|
| Wirkstoff | 1 % |
| Paraffinöl dickflüssig | 10 % |
| Wollfett wasserfrei | 10 % |
| Vaselin weiss | 79 % |
| | 100 % |

Die Bestandteile werden zusammengeschmolzen und steril filtriert.

Präparatebeispiel 8: Zur Insufflation geeignete, 0,025 g 2,4,4'-Trichlor-2'-hydroxydiphenylether enthaltende Kapseln können wie folgt hergestellt werden:

Zusammensetzung: (für 1000 Kapseln)

| | |
|---|---|
| Wirkstoff | 25,00 g |
| Laktose, gemahlen | 25,00 g |

Der Wirkstoff und die Laktose feinst gemahlen werden gut miteinander vermischt. Das erhaltene Pulver wird gesiebt und in Portionen zu je 0,05 g in Gelatinekapseln abgefüllt.

**Patentansprüche**

1. Verwendung einer Verbindung der Formel I

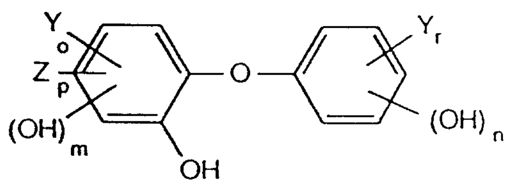

worin

Y Chlor oder Brom,
Z die Reste $SO_2H$, $NO_2$, oder $C_1$-$C_4$-Alkyl und
r die Zahlen 0 bis 3,
o die Zahlen 0 bis 3,
p die Zahlen 0 oder 1,
m die Zahlen 0 oder 1 und
n die Zahlen 0 oder 1

bedeuten,
zur Herstellung pharmazeutischer Präparate für die Behandlung von inflammatorischen, Erkrankungen.

2. Verwendung nach Anspruch 1, dadurch gekennzeichnet, dass in Formel I
Y Chlor oder Brom bedeuten und m = 0, n = 0 oder 1, 0 = 1 oder 2, r = 1 oder 2 und p = 0 sind.

3. Verwendung nach Anspruch 1 oder 2, dadurch gekennzeichnet, dass in Formel I
Y Chlor bedeutet und m = 0, n = 0, o = 1, r= 2 und p = 0 sind.

4. Verwendung nach einem der Ansprüche 1 bis 3, dadurch gekennzeichnet, dass die Verbindung der Formel I in einer Menge von 0,01 bis 15, vorzugsweise 0,1 bis 4 Gew.% im pharmazeutischen Präparat enthalten ist.

5. Verwendung nach einem der Ansprüche 1 bis 4, dadurch gekennzeichnet, dass das pharmazeutische Präparat zusätzlich einen Penetrationsbeschleuniger enthält.

6. Verwendung nach Anspruch 5, dadurch gekennzeichnet, dass das pharmazeutische Präparat als Penetrationsbeschleuniger Harnstoff, Propylenglykol oder Oleinsäure enthält.

**Claims**

1. The use of a compound of the formula I

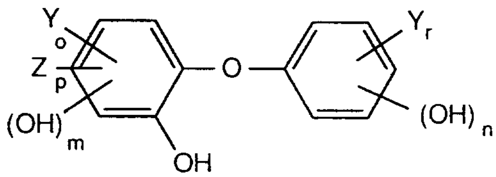

in which

Y is chlorine or bromine,
Z is an $SO_2H$, $NO_2$ or $C_1$-$C_4$alkyl radical,
r is a number from 0 to 3,
o is a number from 0 to 3,
p is the number 0 or 1,
m is the number 0 or 1 and
n is the number 0 or 1

for the preparation of pharmaceutical preparations for the treatment of inflammatory diseases.

2. The use according to claim 1, wherein in formula I Y is chlorine or bromine, m = 0, n = 0 or 1, o = 1 or 2, r = 1 or 2 and p = 0.

3. The use according to either of claims 1 and 2, wherein in formula I, Y is chlorine, m = 0, n = 0, o = 1, r = 2 and p = 0.

4. The use according to any one of claims 1 to 3, wherein the pharmaceutical preparation comprises the compound of the formula I in an amount of 0.01 to 15, preferably 0.1 to 4 % by weight.

5. The use according to any one of claims 1 to 4, wherein the pharmaceutical preparation additionally comprises a penetration accelerator.

6. The use according to claim 5, wherein the pharmaceutical preparation comprises urea, propylene glycol or oleic acid as the penetration accelerator.

**Revendications**

1. Utilisation d'un composé de formule

(I) 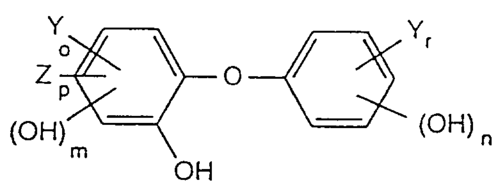

dans laquelle

Y représente un atome de chlore ou de brome,
Z représente un groupe $SO_2H$, $NO_2$ ou alkyle en $C_{1-4}$,
r vaut de 0 à 3,
o vaut de 0 à 3,
p vaut 0 ou 1
m vaut 0 ou 1 et
n vaut 0 or 1,

pour la préparation de compositions pharmaceutiques destinées au traitement de maladies inflammatoires.

2. Utilisation conforme à la revendication 1, caractérisée en ce que, dans la formule(I), Y représente un atome de chlore ou de brome, m vaut 0, n vaut 0 ou 1, o vaut 1 ou 2, r vaut 1 ou 2 et p vaut 0.

3. Utilisation conforme à la revendication 1 ou 2, caractérisée en ce que, dans la formule (I), Y représente un atome de chlore, m vaut 0, n vaut 0, o vaut 1, r vaut 2 et p vaut 0.

4. Utilisation conforme à une des revendications 1 à 3 caractérisée en ce que la préparation pharmaceutique contient le composé de formule (I) à raison de 0,01 à 15 % en poids, de préférence à raison de 0,1 à 4 % en poids.

5. Utilisation conforme à une des revendications 1 à 4, caractérisée en ce que la préparation pharmaceutique contient en plus un agent favorisant la pénétration.

6. Utilisation conforme à la revendication 5, caractérisée en ce que la préparation pharmaceutique contient, comme agent favorisant la pénétration, de l'urée, du propylèneglycol ou de l'acide oléique.